Europäisches Patentamt

European Patent Office

Office européen des brevets

(11) Veröffentlichungsnummer: **0 182 320**
**B1**

(12) **EUROPÄISCHE PATENTSCHRIFT**

(45) Veröffentlichungstag der Patentschrift:
30.08.89

(51) Int. Cl.⁴: **B23K 26/00, C12N 13/00,**
**C12N 15/00, G01N 1/00**

(21) Anmeldenummer: 85114573.0

(22) Anmeldetag: 16.11.85

(54) Verfahren zur Anbringung von Schnitten an biologischem Material.

(30) Priorität: 23.11.84 DE 3442658
15.03.85 DE 3509273

(43) Veröffentlichungstag der Anmeldung:
28.05.86 Patentblatt 86/22

(45) Bekanntmachung des Hinweises auf die Patenterteilung:
30.08.89 Patentblatt 89/35

(84) Benannte Vertragsstaaten:
AT BE CH DE FR GB IT LI NL SE

(56) Entgegenhaltungen:
US-A- 3 670 260

SCIENCE, Band 213, 31. Juli 1981, Seiten 505-512, AAAS,
Lancaster, US; M.W. BERNS et al.: "Laser microsurgery
in cell and developmental biology"

(73) Patentinhaber: BASF Aktiengesellschaft,
Carl-Bosch-Strasse 38, D-6700 Ludwigshafen(DE)

(72) Erfinder: Cremer, Christoph, Prof. Dr.,
Panoramastrasse 67, D-6900 Heidelberg(DE)
Erfinder: Cremer, Thomas, Dr., Weinbrennerstrasse 61,
D-6900 Heidelberg(DE)
Erfinder: Greulich, Karl Otto, Dr., Ploeck 25-27,
D-6900 Heidelberg(DE)
Erfinder: Monajembashi, Shamci, Rahmengasse 5,
D-6900 Heidelberg(DE)
Erfinder: Wolfrum, Juergen, Prof. Dr., Suedring 2,
D-3405 Rosdorf 2(DE)

## Beschreibung

Die vorliegende Erfindung betrifft ein Verfahren zum Bearbeiten von biologischem Material unterschiedlicher Form und Herkunft durch Laserimpulse, die mit einem Lasersystem, bestehend aus einem Pump- und einem Farbstofflaser erzeugt und in einem Mikroskop bis an die theoretisch mögliche Grenze fokussiert werden.

Es ist bekannt, daß Laserlicht, insbesondere das von Farbstofflasern, eine sehr geringe Strahldivergenz hat und daher sehr gut fokussierbar ist. Hierzu genügt es im Prinzip, den Laserstrahl durch eine Linse oder ein System von Linsen auf eine Brennebene abzubilden. Bei der Verwendung von Linsen mit langer Brennweite erhält man einen Fokus, der in seiner Tiefe (in Ausbreitungsrichtung des Lichts) nicht sehr gut definiert ist. Durch Einkopplung in ein Mikroskop kann man dagegen die sehr kurze Brennweite des Mikroskopobjektivs nutzen, um einen Brennfleck geringer Tiefe zu erzeugen, dessen Querschnitt der Wellenlänge des Lichts entspricht.

Eine Vorrichtung der eingangs genannten Art ist aus Science 213 (1981), Seite 505 bis 513, bekannt, wobei durch einen Nd-YAG Laser ein Farbstofflaser gepumpt wurde, dessen Pulse in ein Mikroskop eingekoppelt wurden. Dieses System war bei Pulslängen von 15ns abstimmbar zwischen 217 und 800 nm. Aus den angegebenen Spitzenleistungen von $10^5$ W errechnet sich bei Annahme eines rechteckigen zeitlichen Pulsverlaufs eine Pulsenergie von 1,5 mJ; tatsächlich dürfte wegen der für solche Laser typischen unregelmäßigen Pulsform die Pulsenergie zwischen 0,5 und 0,7 mJ gelegen haben. Neben abstimmbaren Pulsen von 15ns Länge ließ sich die bekannte Vorrichtung bei drei festen Wellenlängen - 266, 532 und 1064 nm - auch mit Pikosekundenpulsen (25ps) betreiben.

Diese Vorrichtung wurde eingesetzt, um in Chromosomen, subzellulären Organellen und Nervenzellen punktförmige Läsionen zu erzeugen. Aus den dadurch bewirkten Ausfallerscheinungen wurden Rückschlüsse gezogen auf die Funktion der bestrahlten Teile.

Laser werden zum Beispiel in der Ophtalmologie zur Fusion von Geweben durch Photokoagulation eingesetzt (A. J. Kavanagh et al., US Patent 3 348 547, siehe auch Koester et al. US Patent 3 670 260). Hierbei werden aber bei stark nichtphysiologischen Temperaturen die zu fusionierenden Teile des Gewebes abgetötet und müssen vom Organismus durch nachwachsendes Gewebe ersetzt werden, wobei es zum Beispiel zu Narbenbildung kommt. Für die Zellfusion ist es aber erforderlich, daß die behandelten Zellen überleben, d.h., es muß ein Fusionsprozeß unter Vermeidung von Koagulation eingeleitet werden.

Der vorliegenden Erfindung lag nun die Aufgabe zugrunde, sehr feine Läsionen, Löcher und äquidistante Schnitte an elongierten biologischen Materialien anzubringen. Generelle Meinung war dabei, daß diese Aufgabe durch quantitativ genau definiertes Absenken der Pulsenergie - verglichen mit den oben angegebenen Pulsenergien - zu lösen sei.

Damit wären Schwelleneffekte so nutzbar, daß Läsionen kleiner als die der Wellenlänge des verwendeten Lichtes erzeugbar wären.

Überraschenderweise ließ sich die gestellte Aufgabe durch einen Schritt in die entgegengesetzte Richtung mit den Merkmalen der Patentansprüche lösen.

Durch Einsatz eines leistungsstarken Excimerlasers und eines ebenfalls leistungsstarken Farbstofflasers konnte die Pulsenergie um eine weitere Größenordnung auf 15mJ pro Puls gesteigert werden. Dadurch wurden Beugungserscheinungen, die theoretisch bei Einkopplung eines Lasers in ein Mikroskop immer zu erwarten sind, und die in der Regel als unerwünschte Nebeneffekte angesehen werden, so stark, daß diese Beugungserscheinungen selbst zum Anbringen von Läsionen benutzt werden konnten.

Das erfindungsgemäße Verfahren basiert also im Wesentlichen auf folgenden Voraussetzungen:

1. Einsatz eines leistungsstarken Pumplasers (z.B. eines Excimer-Lasers).
2. Abstimmbarkeit durch einen nachgeschalteten Farbstofflaser.
3. Einkopplung der Laserstrahlung in ein Mikroskop.
4. Ausnutzung von Beugungserscheinungen.

Diese Erfindungselemente werden im Folgenden genauer beschrieben.

1. Es wird ein Excimer-Laser als Energiequelle verwendet, dessen Wellenlänge im Ultravioletten (UV, 248 und 308 nm) liegt. Damit lassen sich alle verfügbaren Farbstoffe mit hoher Pulsenergie pumpen.
2. Durch Nachschalten eines Farbstofflasers wird das Lasersystem in seiner Wellenlänge abstimmbar. Eine solche Abstimmbarkeit ist für das erfindungsgemäße Verfahren erforderlich
   a) aus physikalischen Gründen, da die Dimensionen des fokussierten Lichts von der Wellenlänge abhängig sind;
   b) aus biologischen Gründen, da unerwünschte Nebeneffekte bei der Bearbeitung von biologischem Material (z.B. Schädigung von genetischem Material) stark von der Wellenlänge abhängig sind.

Da der Farbstofflaser von einem leistungsstarken Excimerlaser gepumpt wird, ist eine kontinuierliche Abstimmbarkeit über einen Wellenlängenbereich von 100 bis 1500 nm gewährleistet. Neben der direkten Anregung von Farbstoffen erlauben die hohen Pumpenergien Frequenzverdopplung, Ramanverschiebung und Frequenzvermischung der Farbstofflaserimpulse.

3. Die vom Farbstofflaser ausgehende Laserstrahlung weist eine sehr geringe Strahldivergenz auf und kann daher bis zur phyikalisch möglichen Grenze fokussiert werden. In dem erfindungsgemäßen Verfahren wird die Fokussierung dadurch erreicht, daß das Licht über den Auflicht-Beleuchtungskanal in ein Mikroskop eingekoppelt und durch das Mikroskop-Objektiv auf den Objektträger des Mikroskops fokussiert wird. Der so entstehende Brennfleck hat wegen der sehr kurzen Brennweite des Objektivs eine sehr geringe Tiefe (Ausdehnung in Strahlrichtung). Somit läßt sich durch Einsatz eines beweglichen Objekttisches und durch Variation der Fokus-Ebene der Brennfleck relativ zum Objekt in drei Dimensionen bewegen. Dies kann z.B. genutzt werden, um mikrochirurgische Eingriffe in biologische Zellen auch in tieferen Zellschichten durchzuführen.

4. Aufgrund von Beugungserscheinungen an den Blendenöffnungen erscheint der Brennfleck in der Brennebene nicht als einfache kreisförmige Scheibe, sondern ist von einem System von Beugungsringen umgeben, deren Abstand voneinander genau der Wellenlänge des verwendeten Lichts entspricht, und die einige Prozente der Gesamtenergie des fokussierten Lichts enthalten. Bei den durch die Kombination von Punkt 1 bis 3 erzielten Pulsen sind die Beugungsringe so stark, daß etwa 5 bis 10 dieser Ringe genutzt werden können, um biologisches Material (wie z.B. Chromosomen) zu schneiden. Da der Abstand der Beugungsringe durch die Wellenlänge des Lichts gegeben ist, lassen sich an länglichen biologischen Objekten exakte Schnitte anbringen. Aufgrund der Abstimmbarkeit des Lasersystems läßt sich der Schnittabstand innerhalb des verfügbaren Wellenlängenbereichs frei wählen. Insbesondere lassen sich wegen der Möglichkeit, auch in UV mit starken Pulsen zu arbeiten, sehr kleine Schnittabstände wählen. Da beim Schneiden mittels Beugungsringen Schwelleneffekte eine Rolle spielen sind die Schnitte sehr fein, mit Schnittstärken wesentlich unterhalb der Wellenlänge des verwendeten Lichts.

Mit der Erfindung wurde es erstmals möglich, feine äquidistante Schnitte an elongiertem biologischen Material anzubringen (s. Abb.), deren Abstand durch die Wahl der Pulsenergie vorgegeben werden kann. Da mehrere Schnitte gleichzeitig gesetzt werden können, und da durch den Einsatz eines berührungsempfindlichen Bildschirms in Verbindung mit einem computergesteuerten Scanning-Tisch schnell neue Objekte in den Strahl geführt werden können, ist zudem die Verarbeitung einer großen Zahl von biologischen Objekten möglich.

Nutzungsbeispiele

1. Zerschneiden von im Lichtmikroskop sichtbaren Chromosomen für Mikrocloning Experimente. Hierdurch läßt sich die Lage bestimmter Gene auf bestimmten Chromosomenabschnitten in einer einfacheren Weise als mit den gängigen molekularbiologischen Verfahren bestimmen.

2. Zerschneiden von Chromosomen aus gesunden Zellen an typischen Bruchstellen, die bei Krebserkrankungen, zum Beispiel beim Burkitt - Lymphom auftreten (in diesem Fall wird lediglich ein Beugungsring genutzt). Solche Experimente können Aufschluß darüber geben, ob Chromosomenbrüche Ursache oder Folge von Erkrankungen sind.

3. Produktion von annähernd monodispersen Oligonukleosomen. Nukleosomen, annähernd scheibenförmige Protein-DNS Komplexe mit einem MW=204000, sind die sich vielfach wiederholenden Bausteine von Chromatin, dem Genmaterial höherer Organismen, das sich während der Zellteilung zum Chromosom zusammenfaltet. Für das Studium solcher Faltungsprozesse, die auch für die Ablesbarkeit von einzelnen Genen von Bedeutung sind, werden idealerweise Chromatinbruchstücke (= Oliginukleosomen) von klar definierter Länge benötigt. Bei derzeit benutzten molekularbiologisch/biochemischen Herstellungsverfahren gelten beispielsweise Bruchstücke mit 40 ± 10 Nukleosomen als "homogen". Bei Einsatz des erfindungsgemäßen Verfahren läßt sich bei einem mittleren Nukleosomabstand von 14nm bei einer Wellenlänge von 56Onm eine deutlich bessere Homogenität (40 ± 1) erreichen.

4. Ähnlich lassen sich intermediäre Filamente, Mikrotubuli oder filamentöse Bakterienphagen zerschneiden mit dem Ziel, die erhaltenen kurzen Stücke dieser grob zylindrischen Strukturen zur Herstellung von Kristallen für Röntgenbeugungsexperimente zu nutzen. Bisher waren für solche elongierten Objekte nur Röntgenbeugunsexperimente an Fasern möglich, die eine wesentlich geringere Information boten. Da bei den in Punkt 3 und 4 beschriebenen Anwendungen die zu bearbeitenden Objekte nicht sichtbar sind, müssen sie in einer Strömung oder in einem elektrischen Felo ausgerichtet und "blind" geschnitten werden.

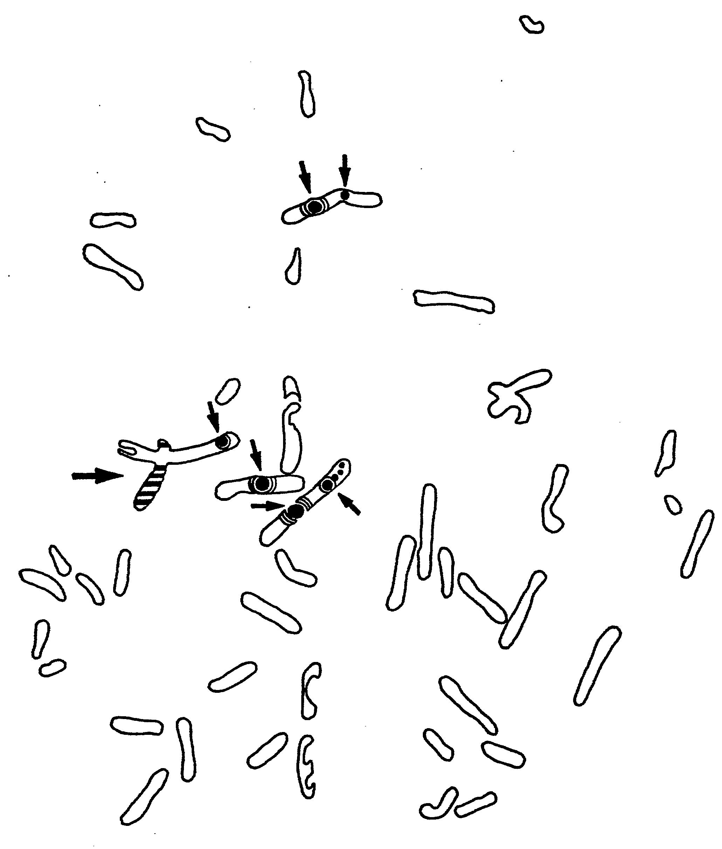

5. Die Abbildung zeigt Chromosomen aus menschlichen Lymphozyten. Einige Chromosomen (kleine Pfeile) sind mit dem gesamten Strahl bearbeitet, so daß sich das gesamte Beugungsmuster in den Läsionen bemerkbar macht. Bei dem durch den großen Pfeil markierten Chromosom wurde das zentrale Beugungsscheibchen außerhalb des Chromosoms angesetzt, so daß nur das annähernd parallele Schnittmuster zu sehen ist. Die verwendete Lichtwellenlänge war 570 nm.

Patentansprüche

1. Verfahren zur Anbringung von Schnitten, Löchern und/oder Mikroläsionen an biologischem Material wie Zellverbänden, Einzelzellen oder Chromosomen durch in ein Mikroskop eingekoppelte Laserpulse, mit folgenden Merkmalen:

a) Einsatz eines leistungsstarken Pumplasers, z.B. eines Eximer-Lasers,

b) Abstimmbarkeit durch einen nachgeschalteten Farbstofflaser,

c) die Laserpulse dauern zwischen $10^{-12}$ und $10^{-3}$ sec,

d) die Laserpulsenergien liegen zwischen $5 \times 10^{-3}$ und $15 \times 10^{-3}$ Joule pro Puls,

e) die Laserwellenlängen liegen zwischen 100 und 1500 nm,

f) die Laserpulse werden bis auf einen durch Beugungserscheinungen begrenzten Querschnitt fokussiert und die Beugungsmaxima n-ter Ordnung (n = 1, 2, 3,...) der Laserpulse zur Anbringung der Schnitte genutzt.

2. Verfahren nach Anspruch 1, wobei die Ausrichtung des biologischen Materials mit Hilfe eines zwei- oder dreidimensional beweglichen computergesteuerten Scanning-Tisches in Verbindung mit einem berühungsempfindlichen Bildschrim, auf dem das Mikrospbild abgebildet wird, geschieht.

## Claims

1. A process for making cuts, holes and/or microlesions in biological material such as cell ensembles, individual cells or chromosomes by means of microscope coupled laser pulses, having the following features:

a) use of a powerful pump laser, for example an eximer laser,

b) tuneability by a downstream dye laser,

c) the laser pulses last from $10^{-12}$ to $10^{-3}$ sec,

d) the laser pulse energies are from $5 \times 10^{-3}$ to $15 \times 10^{-3}$ joule per pulse,

e) the laser wavelengths from 100 to 1500 nm,

f) the laser pulses are focused to a cross-section limited by diffraction phenomena and the diffraction maxima of the n-th order (n = 1, 2, 3,...) of the laser pulses are used for making the cuts.

2. A process as claimed in claim 1, wherein the biological material is aligned by means of a computer controlled scanning table which is mobile in two or three dimensions in combination with a touch sensitive screen depicting the microscope image.

## Revendications

1. Procédé pour pratiquer des coupes, trous et/ou microlésions sur des matériaux biologiques tels que des associations de cellules, cellules isolées ou chromoses, par des impulsions laser introduites dans un microscope, avec les caractéristiques suivantes:

a) emploi d'un laser de pompage forte puissance par exemple un laser Eximer,

b) possibilité d'accord par un laser couleur branché à la suite,

c) les impulsions laser durent entre $10^{-12}$ et $10^{-3}$ s,

d) les énergies d'impulsion laser sont comprises entre $5 \times 10^{-3}$ et $15 \times 10^{-3}$ joule par impulsion,

e) les longueurs d'onde laser sont comprises entre 100 et 1500 nm,

f) les impulsions laser sont focalisées jusqu'à une section limitée par des phénomènes de diffraction et le maximum de diffraction de l'ordre n-ter (n = 1, 2, 3,...) des impulsions laser est utilisé pour pratiquer les coupes.

2. Procédé selon la revendication 1, dans lequel l'arrangement du matériau biologique apparaît à l'aide d'une table de Scanning commandée par ordinateur et mobile suivant deux ou trois dimensions, en liaison avec un écran sensible au contact sur lequel se forme l'image du microscope.

EP 0 182 320 B1